(19) Europäisches Patentamt / European Patent Office / Office européen des brevets

(11) **EP 3 920 191 A1**

(12) **EUROPEAN PATENT APPLICATION**

(43) Date of publication:
08.12.2021 Bulletin 2021/49

(51) Int Cl.:
*G16H 50/20* (2018.01)  *G16H 50/30* (2018.01)

(21) Application number: **20178631.6**

(22) Date of filing: **05.06.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Sensyne Health Group Limited Oxford OX4 4GE (GB)**

(72) Inventors:
• **ANDREOTTI LAGE, Fernando**
**Oxford, OX4 4GE (GB)**

• **DÜRICHEN, Robert**
**Oxford, OX4 4GE (GB)**
• **HELDT, Frank Stefan**
**Oxford, OX4 4GE (GB)**
• **CARR, Oliver Sebastian Leslie**
**Oxford, OX4 4GE (GB)**
• **JOVANOVI , Stojan**
**Oxford, OX4 4GE (GB)**
• **KHAN, Rabia Tahir**
**Oxford, OX4 4GE (GB)**

(74) Representative: **Korenberg, Alexander Tal et al Kilburn & Strode LLP Lacon London 84 Theobalds Road London WC1X 8NL (GB)**

(54) **MACHINE LEARNING METHOD AND SYSTEM SUITABLE FOR PREDICTION OF DISEASE ONSET USING MULTI-VARIATE LONGITUDINAL PATIENT DATA**

(57)    A computer-implemented method of training a neural network to predict a disease onset label corresponding to a diagnostic event of a disease, the method comprising providing training data comprising input data and target data for a plurality of patients comprising target patients having a diagnostic event of the disease and control patients not having a diagnostic event of the disease, the input data comprising a set of data values of respective variables for each of a plurality of patients, wherein the set comprises for each variable one or more data values associated with respective one or more observation periods, and the target data comprising a disease onset label for each of the plurality patients, wherein each disease onset label indicates whether a respective patient has a diagnostic event of the disease that occurs within each of two or more time periods; and training, using the training data, a neural network model to predict a corresponding disease onset label from the respective variables associated with one or more observation periods, the disease onset label indicating whether a respective patient has a diagnostic event of the disease that occurs within each of the two or more time periods. A computer-implemented method of predicting a disease onset label corresponding to a diagnostic event of a disease, the method comprising providing test input data comprising a set of data values of respective variables associated with one or more observation periods for a test patient and applying the test input data as an input to a neural network trained according to claim 1 to predict a disease onset label indicating the likelihood of the test patient having a diagnostic event of the disease that occurs within each of the two or more time periods.

100

receive and preprocess training data — 102

use training data to train a neural network model to predict disease onset — 104

predict disease onset for two or more time periods for a test patient — 106

Fig. 1

**Description**

**TECHNICAL FIELD**

The present disclosure relates to a machine learning system and method suitable for prediction of the onset of diseases using multi-task learning, and in particular, although not exclusively, using recurrent neural networks, for example for predicting the onset of cardiovascular diseases based on electronic healthcare records (EHR).

**BACKGROUND**

The prediction of disease onset is a fundamental step in implementing preventive interventions and easing burden on national health systems. Multi-variate longitudinal patient data have become ubiquitous, with hospitals routinely collecting data covering a large number of variables over many years. The data collected contains longitudinal patient information on demographics, vital signs, laboratory results, medications, diagnoses and procedures. Recent studies indicate that by making efficient use of EHRs, improved risk prediction models can be developed for different diseases, which would lead to better and more cost-effective treatments (see Goldstein, Benjamin A., et al. "Opportunities and challenges in developing risk prediction models with electronic health records data: a systematic review." Journal of the American Medical Informatics Association 24.1 (2017): 198-208, incorporated by reference in this disclosure).

However, the analysis required in order to improve the prediction of disease onset remains challenging due to high dimensionality, noise, heterogeneity, sparsity, random errors during data collection and systematic biases in the patient data. As pointed out by [9], published algorithms are primarily based on linear models, such as logistic or Cox regression, and use only a small number of features. Given the recent advances in machine learning, there is a growing interest in developing disease onset prediction algorithms using architectures such as neural networks, which have been shown to work well with high-dimensional noisy data.

There remains a need in the art for a method of reliably and accurately predicting disease onset for multiple time periods to allow hospitals and clinicians to assess the risks of a disease simultaneously and efficiently using large rich datasets.

**SUMMARY**

A computer-implemented method of training a neural network to predict a disease onset label corresponding to a diagnostic event of a disease is provided. The method comprises providing training data comprising input data and target data for a plurality of patients comprising target patients having a diagnostic event of the disease and control patients not having a diagnostic event of the disease. The input data comprises a set of data values of respective variables for each of a plurality of patients, wherein the set comprises for each variable one or more data values associated with respective one or more observation periods, and the target data comprising a disease onset label for each of the plurality patients. The disease onset label indicates whether a respective patient has a diagnostic event of the disease that occurs within each of two or more time periods.

The method comprises training, using the training data, a neural network model to predict a corresponding disease onset label from the respective variables associated with one or more observation periods, the disease onset label indicating whether a respective patient has a diagnostic event of the disease that occurs within each of the two or more time periods.

A computer-implemented method of predicting a disease onset label corresponding to a diagnostic event of a disease, the method comprising providing test input data comprising a set of data values of respective variables associated with one or more observation periods for a test patient and applying the test input data as an input to a neural network trained according to the method described above to predict a disease onset label indicating the probability or a confidence score of the test patient having a diagnostic event of the disease that occurs within each of the two or more time periods. In relation to the output of the neural networks, at least when a soft-max or sigmoid activation function is used, the terms probability and confidence score are used interchangeably herein. Predictions of classification outputs can be obtained by comparing the outputs of different units, by thresholding or in any other suitable way.

Advantageously, by training the model to predict one of a number of time periods of disease onset for each patient, the model is trained with patients having had a disease onset timing corresponding to each of the time periods. By contrast, if a separate model would be trained for each time period, then at least some of the time periods would be trained on less than the total number patients available for training. For example, a model for a time period of less than one month would have less patients to train on than a model for a time period of less than a year. By making a prediction amongst a number of time periods (for example less than one month or less than one year), the disclosed model can be trained to make predictions about multiple time periods using all the patients for all the time periods. In this sense, information is in effect shared between the training for time period(s) with fewer patients associated and the total patient population used in training. This is particularly beneficial if the number of patients available for training for any one time period is limited.

What is more, by training one model to predict several time periods of disease onset, rather than a separate respective model for each time period, the number of models to be trained is reduced at least by half, providing a corresponding reduction in the computational resources (processor cycles, memory allocation) required to make predictions amongst multiple time periods of disease onset. For example, where all models are trained for the same number of epochs, the number of training epochs is reduced at least by half and where the individual models are of similar computational complexity, the training time can also be expected to be reduced by at least half. The reduction in resources is even greater if more than two time periods are considered in training and prediction, for example three or four time periods.

Another way in which the training of the same model for multiple time periods can be beneficial is that it provides more granular training information in terms of the target data in some circumstances, for example in the case of overlapping time periods. In one specific example, consider a number of time periods starting at a common reference point and having different end points (for example some or all of 1, 3, 6, 12, 24 or more than 24 months from the reference point). In that case, in all but the first time period, the target data is more fine-grained and detailed in the case of training one model on all the time periods as opposed to training each model separately. For example, training the models for 3, 6, 12, 24 and more than 24 months time periods individually would group all targets together with the same target data (disease onset or not within the relevant time period), while when trained together patients would have different target data within each time period depending on which of the shorter time periods their disease onset falls. For example, in the multiple time period training disclosed in this application a patient with a 23 month disease onset would have different target data than a patient with an 11 month disease onset. By contrast, in a single time period model training for the 24 months period, both of these would have the same target datum ("developed disease within 24 months"). It is believed that this more granular target data can improve the robustness of training and lead to more consistent prediction accuracy across training data sets.

The observation period may be one day, for example a day spent in a hospital during which measurements were taken and data collected that are then used to obtain, directly or by computation, the data values of the variable. The observation period may be a plurality of days or an entire hospital admission over one or more days. In some embodiments, an observation period may be less than a day. In either case, the data values are obtained directly or by computation from data collected during the observation period. The collected data may be data collected during a hospital admission of a respective patient but equally, the collected data may be primary or secondary care data, nursing or care home data, out-of-hospital observations or any other type of patient data. The hospital admission may refer to admission types covering but not limited to inpatient, outpatient or emergency department admissions. It will be understood that the set of data values may not be complete and that data values may be missing and not be available for all of the variables and observation periods. For example certain variables may have available data values for only some but not all of the observation periods.

The time periods may, for example, all start at a common reference point and each have a different respective end point in time. In one such example, the time periods may, for example, be: 1 months from the reference point; 3 months from the reference point; 12 months from the reference point, over 12 months from the reference point and so forth.

Providing the training data may comprise obtaining the input data from health records. The health records may be electronic health records. The training data may comprise two or more categories of the categories of: demographic information; admission information; diagnoses; procedures; laboratory test values; prescribed medications; observations of vital signs; and demographic or anthropomorphic measurements. The training data may comprise three or more categories from the list of categories. The training data may comprise four or more categories from the list of categories. The training data may comprise five or more categories from the list of categories. The training data may comprise six or more categories from the list of categories. The training data may comprise seven or more categories from the list of categories. The training data may comprise all categories from the list of categories.

The neural network model may be a recurrent neural network. The recurrent neural network may be a gated recurrent unit (GRU) neural network, a recurrent neural network with attention mechanism, a bidirectional neural network or a combination of these.

Training the recurrent neural network may comprise applying the data values for the respective variables for each of the plurality of patients as input to the recurrent neural network sequentially, one set of data values for the respective variables associated with one observation period at a time, and reducing a loss, for example a classification loss, across the data values. The recurrent neural network may predict a disease onset label for a respective patient by applying the data values for the respective variables for the respective patient as input to the recurrent neural network sequentially to produce an output for the respective patient.

The disease may be a cardiovascular disease. The cardiovascular disease may be ischaemic stroke or myocardial infarct.

Aspects extend to one or more computer-readable media encoding computer instruction that, when executed on a computing device, implement methods as described above and to a system comprising the one or more computer readable media, a memory for storing the artificial neural network and the data units and a processor for executing the instructions.

The present disclosure refers to artificial neural networks. It will be appreciated that artificial neural networks, for example a recurrent neural network, represent a specific parametrization of a non-linear function (in terms of network weights) and that the present disclosure is not limited by the language used to describe the non-linear function or its structure. It will be understood that an artificial neural network in the context of a computer implemented invention refers to a physical entity that exists either in terms of a physical state of a general purpose or specifically adapted computing platform or in a specifically adapted physical circuitry, for example.

## BRIEF DESCRIPTION OF THE FIGURES

Illustrative implementations of the present disclosure will now be described, by way of example only, with reference to the drawings. In the drawings:

**Figure 1** schematically depicts a process of predicting a disease onset label corresponding to a diagnostic event of a disease of interest using a neural network model;

**Figure 2** shows an example of a training data instance for a patient p;

**Figure 3** shows the architecture of a gated recurrent unit;

**Figure 4** shows a schematic of a neural network model;

**Figure 5** shows a schematic of unidirectional and bidirectional recurrent neural networks;

**Figure 6** shows a schematic of a further neural network model;

**Figure 7** shows an example of an aggregated feature vector containing the data values for all the observation periods and a feedforward neural network for predicting disease onset for three time periods;

**Figure 8** shows a table with the number of patients in the two detailed examples (stroke and MI cohorts);

**Figure 9** shows a table with the average area under the receiver operating characteristic curve (AUC) for the multi time period GRU network (MT-GRU) and benchmark models for the prediction of myocardial infarct (MI);

**Figure 9A** shows the equivalent bar charts and error bars for the results in Figure 9;

**Figure 10** shows a table with the average area under the receiver operating characteristic curve (AUC) for the multi time period GRU network (MT-GRU) and benchmark models for the prediction of ischaemic stroke;

**Figure 10A** shows the equivalent bar charts and error bars for the results in Figure 10; and

**Figure 11** illustrates a block diagram of one implementation of a computing device.

## DETAILED DESCRIPTION

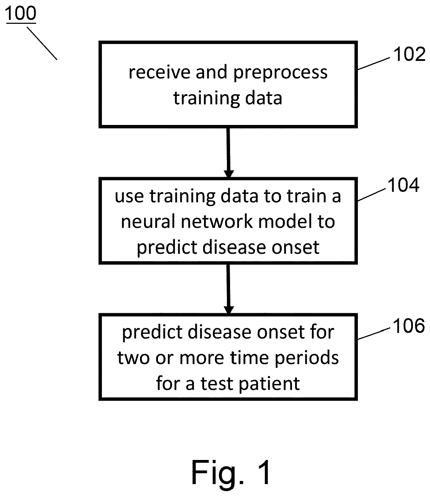

Figure 1 schematically depicts a process 100 of predicting a disease onset label corresponding to a diagnostic event of a disease of interest using a neural network model. The disease onset label indicates whether a patient will have a diagnostic event of the disease that occurs within each of two or more time periods. In some embodiments, the disease of interest may be a cardiovascular disease such as ischemic stroke or myocardial infarction.

The process comprises a step 102 of providing training data comprising input data and target data for a plurality of patients. The plurality of patients comprise target patients having had a diagnostic event of the disease and control patients not having had a diagnostic even of the disease. Each target patient is matched with a corresponding control patient in the input data. By way of non-limiting example, the target patients can be matched to control patients using a nearest-neighbour matching approach on the corresponding patients in the control cohort.

The input data comprises a set of data values of variables for each one of the plurality of patients. For each patient, each data value is associated with a respective observation period during which the data value, or values used to compute the data value, for example by averaging, was collected. For each variable and each patient there are one or more data values associated with a respective observation period each. In some embodiments, the observation period is a single day, for example a day spent in a hospital during which measurements were taken and data collected that are then used to obtain, directly or by computation, the data values of the variable. In other embodiments, the observation period may be a plurality of days or an entire hospital admission over one or more days. In some embodiments, an observation period may be less than a day. In either case, the data values are obtained directly or by computation from data collected during the observation period. The collected data may be data collected during a hospital admission of a respective patient but equally, the collected data may be primary or secondary care data, nursing or care home data, out-of-hospital observations or any other type of patient data. The hospital admission may refer to admission types covering but not limited to inpatient, outpatient or emergency department admissions. It will be understood that the set of data values may not be complete and that data values may be missing and not be available for all of the variables and observation periods. For example, certain variables may have available data values for only some but not all of the observation periods.

In some embodiments, the input data may comprise data from electronic health records (EHR). The input data for each patient comprises data values of variables associated with at least one observation period. The input data, for example derived from EHR, may contain demographic information (e.g sex, age, ethnicity), admission information (e.g. start/end dates, discharge method, discharge destination, admission type - in-patient, outpatient, emergency department etc), diagnoses (e.g. ICD-10 codes such as J90.X - Pleural effusion, not elsewhere classified, H53.4 - Visual field defects), procedures (e.g. OPCS4 coded such as U19.2 - 24 hour ambulatory electrocardiography, Z50.1 - skin of arm), laboratory test values (e.g. Laboratory Information Management System (LIMS), blood and urine tests such as blood cholesterol (HDL ratio), blood albumin, blood haemoglobin), prescribed medications (e.g. prescribed both during visits and take-home, BNF codes such as 05.01 - antibacterial drugs, 01.03 -antisecretory), observations of vital signs (e.g. systolic blood pressure, temperature, heart rates) or anthropomorphic measurements (e.g. BMI, weight, height, deprivation scores, smoking status). The input data may also comprise variables indicating the existence or absence of comorbid conditions or variables indicating an overall comorbidity score such as Charlson's comorbidity score (see Quan, Hude, et al. "Updating and validating the Charlson comorbidity index and score for risk adjustment in hospital discharge abstracts using data from 6 countries." American journal of epidemiology 173.6 (2011): 676-682, incorporated by reference in this disclosure), computed as the sum of weights associated with comorbid conditions . It will be appreciated that the data may be obtained from non-electronic health records by suitable processing including for example but not limited to optical character recognition, natural language processing, human operator input and the like.

Subsequent to obtaining the input data, step 102 may in some embodiments comprise preprocessing the input data or the input data may be received already preprocessed or may be used without preprocessing. The preprocessing may include but is not limited to one or more of the following: data cleaning, data aggregation, data imputation, data scaling, exclusion criteria. Data cleaning may comprise standardizing data coming from multiple trusts by using standard medication codes such as BNF (British National Formulary), standardising laboratory result names and values, removing non-numeric entries. Data aggregation may comprise, for each one of the plurality of patients, aggregating daily data values of variables into a feature vector for each patient and all observation periods or into a set of feature vectors, each feature vector corresponding to an observation period, for each patient. By way on non-limiting example, continuous variables such as laboratory test values or vital signs may be aggregated by taking the median and/or median absolute deviations (or mean and/or standard deviation or any suitable measures) over multiple measurements in the observation period, while categorical variables may be one-hot-encoded (e.g. diagnoses or procedures) or counted (e.g. medications). Data imputation may comprise imputing a fixed value or the populational mean for missing values. Data scaling may comprise standardising features. Further exclusion criteria may be applied such as removing patients without any data values beyond a specific time scale prior to the event of the specific disease.

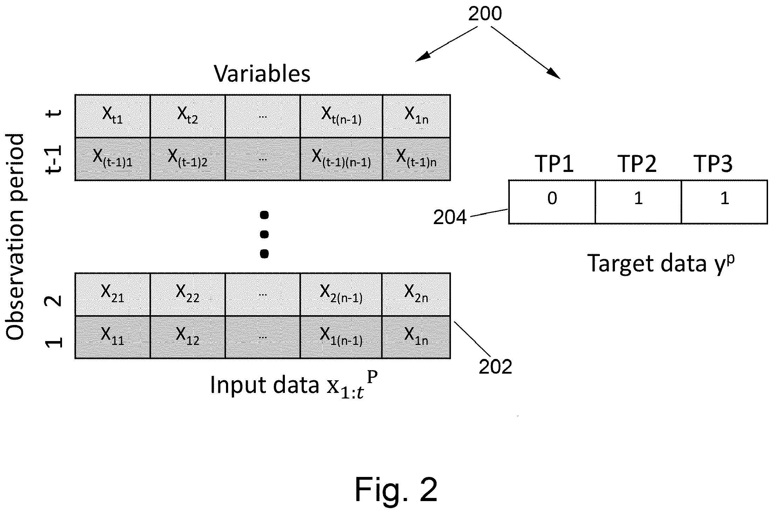

In some embodiments, each patient $p$ is represented in the input data by a plurality of feature vectors $x_i^P$ corresponding to the one or more observation periods $i \acute{e}\{1,...,n\}$. Therefore, each patient p has a corresponding entry in the input data represented by a sequence of feature vectors $x_{1:t}^P$, for $t$ observation periods. Figure 2 shows an example of training data 200 for a patient $p$ with input data $x_{1:t}^P$ 202 for $t$ observation periods and target data 204.

In some embodiments, the GRU neural network is implemented so that each patient's sequence of feature vectors $x_{1:T}^P$ has a fixed number of observation periods $N_{pad}$. If a patient's sequence of feature vectors $x_{1:T}^P$ has less than $N_{pad}$ number of observation periods ($T < N_{pad}$), zero padding (with a required number of zero input vectors $x_t = [0,0,... ]$) is used to pad the sequence of features vectors $x_{1:T}^P$ to $N_{pad}$ number of observation periods. If a patient patient's sequence of feature vectors $x_{1:T}^P$ has more than $N_{pad}$ number of observation periods ($T > N_{pad}$), then $x_{1:T}^P$ is truncated to $N_{pad}$ feature vectors $x_t$ corresponding to $N_{pad}$ observation periods. In other embodiments, the GRU neural network is implemented so that the number of observation periods can vary from patient to patient.

The target data comprises a disease onset label for each of the plurality of patients. The disease onset label indicates whether a respective patient has a diagnostic event of the disease that occurred within each of two or more time periods. The time periods may be taken relative to a common reference point in time, for example a last hospital admission or other event. The time periods may be separate. In one such example, the time periods could for example be: less than 6 months from the reference point; between 6 to 12 months from the reference point; between 12 to 24 months from the reference point and so forth. In some cases, the time periods may be partially overlapping. The time periods may, for example, all start at the common reference point and each have a different respective end point in time. In one such example, the time periods may, for example, be: 6 months from the reference point; 12 months from the reference point; 24 months from the reference point and so forth. The target data for a patient p is represented by a vector $y^P$ formed of a plurality of binary variables, each binary variable corresponding to a distinct time period from the two or more time periods. Figure 2 also shows an example of a target data $y^P$ 204 for a patient $p$. In this example, the target data is a vector or length three in which the first element is equal to 0 and indicates that patient $p$ has not had a diagnostic event of the disease that occurred in the first time period TP1(for example within less than 6 months). The last two elements of the vector have a value of 1 and indicate that patient $p$ has had a diagnostic event that occurred in the second and third time periods, TP2 and TP3 (for example within less than 12 months and within less than 24 months, respectively). The time period may be expressed in days, months, years, hours or any other suitable unit or mixture of units of time appropriate to the application at hand.

Subsequent to obtaining the training data at step 102, at step 104, the training data is used to train a neural network model to predict a disease onset label from the data values associated with one or more observation periods. The disease onset label predicted by the machine learning model indicates whether a respective patient has a diagnostic event that occurs within each of the time periods defined at step 102.

In some embodiments, the neural network model comprises a recurrent neural network model. A recurrent neural network is a class of artificial neural networks where connections between nodes form a directed graph along a temporal sequence. Recurrent neural networks can use their internal state, also called memory, to process the sequences provided as input. Basic recurrent neural networks comprise a network of neurons (or nodes or units) organized into successive layers. Each node in a given layer is connected with a directed connection to one or more nodes in the next successive layer. Each node has a time-varying real-valued activation, while each connection has a modifiable real-values weight. Nodes are either input nodes receiving input data (such as the vector xf described above), output nodes outputting results (such as $y^P$) or hidden nodes that process the input data and pass it on to the output nodes.

For a supervised learning task (i.e. a learning task in which the target data are available) in discrete time sequences, the input data arrives at the input nodes, one input vector at a time. At any given time step, each non-input unit computes its current activation (result) as a nonlinear function of the weighted sum of the activations of all units that connect to it. During training, the target data is supplied for one, some or all output units at certain time steps. For example, if the input sequence is the $x_{1:T}^P$ for a patient $p$ and $T$ observation periods and the patient had a disease diagnostic event that occurred within 100 days, the final target data at the end of the sequence may be a disease onset label $y^P$ that indicates that the diagnostic event occurs within the time periods in $y^P$ that fit the 100 day criteria (for example within a 3 or 6 month time period, but not within a one month time period).

Each instance of the input data produces an error computed as the deviation of the output of the recurrent neural network from the corresponding target data. During training, a recurrent neural network aims to minimize this error for all instances of the input data and this way the recurrent neural network learns to produce outputs that are close to the target data. Depending on the task, there are different mathematical expressions, known as cost function, loss function, etc, which compute the deviation of the output of the recurrent neural network from the corresponding target data. Recurrent neural networks are trained using backpropagation, which will be described later.

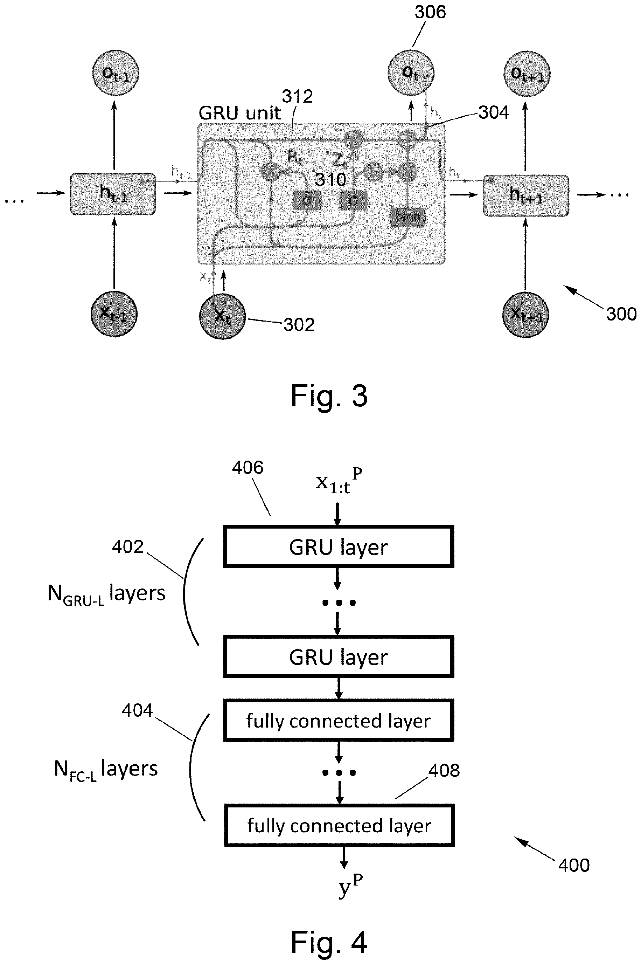

In some embodiments, the recurrent neural network model may be a gated recurrent unit (GRU) neural network. A GRU neural network is a specific type of a recurrent neural network that uses GRU cells, which feature specific gating mechanisms. Gating mechanisms are widely used in recurrent neural network models, where they allow to retain and erase memory from previous sequence steps, and gradients to backpropagate more easily through depth or time Figure 3 illustrates schematically the specific gating mechanism of a GRU neural network. The GRU architecture 300 in Figure 3 is described by the following equations:

$$Z_t = \sigma_g(W_z x_t + U_z h_{t-1} + b_z) \,(1)$$

$$R_t = \sigma_g(W_r x_t + U_r h_{t-1} + b_r) \,(2)$$

$$h_t = Z_t \odot h_{t-1} + (1 - Z_t) \odot \Phi_h(W_h x_t + U_h(R_t \odot h_{t-1}) + b_h \,(3)$$

where $x_t$ 302 is the input vector, $h_t$ 304 is the hidden state vector, $o_t$ 306 is the output vector, $Z_t$ 310 is the update gate vector and $R_t$ 312 is the reset gate vector. The $W$ and $U$ symbols in the equations denote the weight matrices, and $b$ denotes bias terms. Lastly, $\sigma_g$ denotes the sigmoid function, $\Phi_h$ denotes the hyperbolic tangent function and the operator $\odot$ denotes the Hadamard product.

For example, when training a GRU neural network to predict the disease onset label for using the training data from step 102, the input vector $x_t$ 302 in Figure 3 may contain the input patient data from step 102. The GRU neural network takes as input the aggregated patient information for each patient $p$ as a sequence of $t$ observation periods $x_{1:t}^P$, as illustrated in Figure 3. Next, each feature vector $x_t$ in sequence $x_{1:t}$ is processed by the gated recurrent unit according to equations (1), (2), (3) described above.

Figure 4 shown an example of a GRU neural network 400 formed of $N_{GRU-L}$ such GRU layers 402 followed by $N_{FC-L}$ fully connected layers 404. Each layer has $N_{units}$ hidden units. The prediction of the GRU neural network is computed by passing the activations of the final fully connected layer through a sigmoid activation function. In some embodiments, a recurrent L2 regularization with weight $w_{L2}$ may be applied to each GRU layer 406. In some embodiments, the activation function for the GRU hidden state outputs is a rectified linear unit function and the GRU is initialized using a Glorot uniform intialisation. In some embodiments, layer normalization is applied after an activation, followed by a dropout layer with probability $p_{dropout}$. The optimal values of the network's hyperparameters, such as the ones described here, vary according to the complexity and size of the training set and are optimized during training. The hyperparameters of a neural network refer to one or more of variables which determine the network structure (e.g. number or layers, number of hidden units), the variables which determine how the network is trained (e.g. learning rate, weight initialization). In some embodiments, a Bayesian optimization scheme is applied to search for the optimal values, as detailed later. It will be appreciated that different initialisations, activation functions and regularization functions may be used in dependence upon the application and as found suitable, by trial and error or otherwise, all of which will be readily apparent to the person skilled in the art.

The final fully connected layer 408 of the GRU neural network 400 comprises $N_{nodes}$ nodes, where $N_{nodes}$ is equal to the total number of time periods (elements of the target data vector $y^P$) to be predicted. Each node of the final fully connected layer 408 corresponds to one element of the target data vector $y^P$. As explained above, each element of the target data y corresponding to one time period for which the probability of the disease onset is predicted. By using the target data y, the GRU neural network 400 is trained to predict for each patient $p$ the probability $y^P \in [0,1]$ of a diagnostic event of the disease for each of the two ore more time periods defined in the target data. Each output neuron of the final fully connected layer is considered as a separate random binary variable.

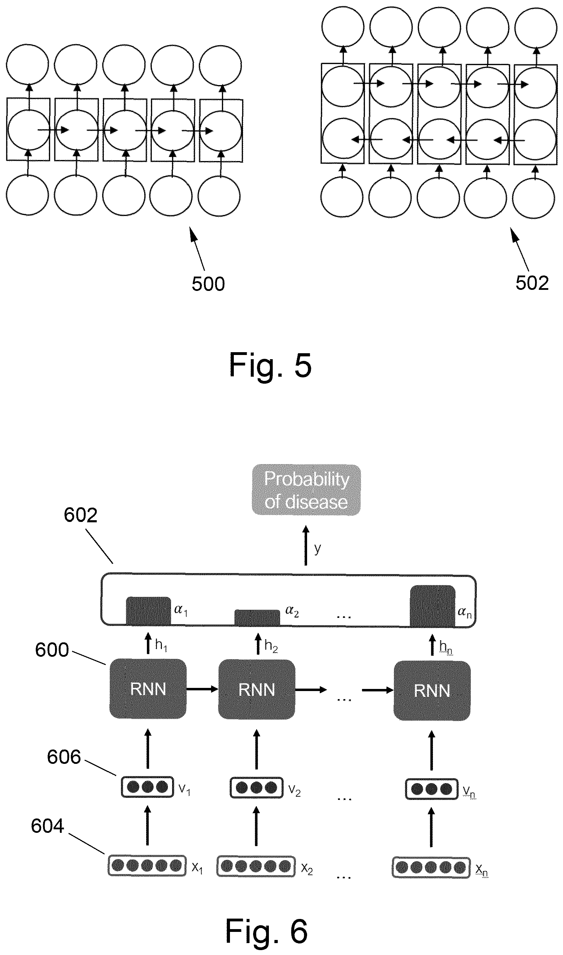

In some embodiments, the recurrent neural network model may be a bidirectional recurrent neural network. The principle of bidirectional recurrent neural networks is to split the neurons of a regular recurrent neural network into two directions, one for positive time direction (forward states), and another for negative time direction (backward states). The general structure of a unidirectional recurrent neural network 500 and a bidirectional recurrent neural network 502 is depicted in Figure 5. A bidirectional recurrent neural network layer connects two layers of opposite directions (i.e. positive and negative time directions) to the same output. The outputs of each state are connected to the inputs of the opposite direction states. This structure allows the network to have both backward and forward information about the input sequence at every time step. Since updating the input and output layers cannot be done at once, the training procedure for a bidirectional recurrent neural network is as follows (using the notations in Figure 3). Firstly, during the forward pass, the forward states ($h_1$, $h_2$, ... , $h_t$) and backward states ($h_t$, $h_{t-1}$ ..., $h_1$) are passed first, then the output neurons ($o_1$, $o_2$, ..., $o_t$) are passed. Secondly, during the backward pass the output neurons ($o_t$) are passed first, then forward states ($h_1$, $h_2$, ... , $h_t$) and backward states ($h_t$, $h_{t-1}$ ..., $h_1$) are passed after. After the forward and the backward passes are completed, the weights (i.e. *W, U, b*) are updated.

In some embodiments, the recurrent neural network may be implemented with an attention mechanism. Attention mechanisms allow neural networks to focus on specific parts of the input data, while neglecting others. In recurrent neural networks, attention is typically implemented by assigning weights to each step of the input sequence when making a prediction, which increases the importance of some steps over others (Bahdanau et al., 2014). To find the best weights to assign to each step, the input sequence is aligned with the output using an alignment model. Several alignment models are well-known in the art. One of the most efficient alignments uses the dot product between input and output and is known as multiplicative or Luong attention (Luong et al., 2014). In some embodiments, Luong attention is used to implement the attention mechanism. As illustrated in Figure 6, the recurrent neural network 600 encodes the input data at each step into hidden states ($h_1$, $h_2$, ... , $h_t$) and the attention layer 602 assigns weights ($\alpha_1$, $\alpha_2$, ..., $\alpha_t$) to each observation period of a patient's input data. Instead of only using the final hidden state ($h_t$) to make a prediction, the attention mechanism calculates a weighted average of all hidden states ($\alpha_1 h_1 + \alpha_2 h_2 + \cdots + \alpha_t h_t = o_t$). Besides the alignment model, the attention layer 602 also includes an activation function. In some embodiments, the activation function used for the attention layer 602 is the softmax function. The softmax function ensures that each hidden state $h_t$ is assigned a weight between 0 and 1, and that the sum of all attention weights is 1. The attention layer 602 is trained together with the rest of the neural network model 600, which results in the attention weights focusing on the observation periods from a patient's input data that are most useful for predicting the disease onset label.

Optionally, the input data $x_{1:t}^P$ can be embedded into a lower dimensional representation $x_{1:t}^P$, using a dimensionality reduction approach such as PCA or an autoencoder neural network, and the lower dimensional representation is then used as an input to the RNN. This optional step 606 is also shown in Figure 6, but it will be appreciated that this can, in some embodiments, equally apply to any of the neural network models discussed in this disclosure.

The neural network model at step 104 is trained to predict disease onset for two or more time periods, each considered as a separate binary variable. The benefit of this approach is that the neural network model can be trained using all the available training data without having to separate the subjects into different training groups based on the onset time period. For example, given the target vector 204 $y^P=[0,1,1]$ in Figure 2 for a first, second and third time periods (TP1=6 months, TP2= 12 months, TP3=24 months), a neural network model would be trained to predict disease onset for all three time periods using the all training data. By contrast, a separate model predicting disease onset for TP1 would only be trained using a subset of the training data (i.e. only patients with disease onset <12 months). As a result, training is more efficient in the case of the model predicting disease onset for two or more time periods, as the whole training dataset can be used and the information contained in the entire dataset can be used for training to predict each of the two or more time periods. This is particularly beneficial if the training data contains only a limited number of patients available for training for any one time periods, as it might be the case with particularly short time periods such as TP1 or even shorter. Not only this is more efficient as the training data does not need to be divided into smaller datasets but the sharing of information between different time periods can lead to better training results, as illustrated by the results in the Detailed Example section below.

Another benefit of the approach is that, by training one model to predict several time periods of disease onset rather than separate models for each time period, the number of models to be trained is reduced at least by half. This provides a corresponding reduction in computational resources (e.g. processor cycles, memory allocation) required to make predictions amongst multiple time periods of disease onset. The reduction is even greater if more than two time periods are used. For example, training three separate neural network to predict disease onset for each of TP1, TP2 and TP3 would use three times more such computational resources when compared to training a single neural network model to predict disease onset for all three time periods simultaneously. This benefit is covered in more detail in the Detailed Example section where we show that the total number of epochs required to train one model to predict several time period or disease is reduced at least by half when compared with training separate models for each time period.

A further benefit of the approach is the provision of more granular training information in terms of the target data in some circumstances as, for example, in the case of overlapping time periods. For example, a patient with 23 month disease onset would have different target data ($y^P$=[0,0,1]) than a patient with a 11 month disease onset ($y^P$=[0,1,1]) when a neural network model is trained to predict the likelihood of disease onset occurring in the three time periods TP1, TP2, TP3. By contrast, in a single time period model, training for the TP3=24 months period, both of these would have the same target datum ("developed disease within 24 months"). Training a neural network model using more granular target data can improve the robustness of training and lead to more consistent prediction accuracy across training data sets.

The neural network model considers each output neuron of the final fully connected layer as a separate random binary variable, and the loss function for the target vector y is the average of the loss of the single binary variables in the target vector. In some embodiments, the loss of the single binary variables is defined as binary cross entropy and therefore the loss function of the neural network model becomes:

$$binary\ cross\ entropy\ loss\ function\ L\ = \frac{-1}{C} \sum_{c=1}^{C} y_c \log(\widehat{y_c}) + (1 + y_C)\log(1 - \widehat{y_c})$$

where $c$ is the time period for which the probability is predicted, $y_c$ is the variable in the target data corresponding to time period $c$, and $\widehat{y_c}$ is the predicted data. The predictions take values between 0 and 1 for each observation period in the target data.

In some embodiments, the hyperparameters of the network are optimized using Bayesian optimization. Bayesian optimization is a method for global optimization where the objective function $f(x)$ that is optimized is a black box-function that does not assume any functional forms. Bayesian optimization is used to maximise the area under the receiver operating characteristic curve (AUC) of the model. Specifically, a set of hyperparameters are chosen, the model with these parameters is trained, and predictions are made using the input data from which the AUC is calculated. The process is repeated and the hyperparameters that produce the highest AUC are chosen. The hyperparameters optimized using Bayesian optimization include the number of recurrent layers (e.g. $N_{GRU-L}$ and $N_{FC-L}$ above), the number of hidden units in each layer, the number of observation periods $N_{pad}$, the dropout rate $p_{dropout}$, the learning rate $L_{rate}$ and the L2 regularisation weight.

In some embodiments, the neural network model comprises a regular, non-recurrent, feedforward neural network model, for example a densely connected neural network. A feedforward neural network is formed of an input layer, one or more hidden layers and an output layer. Each layer is formed of one or more elementary units known as neurons and which can implement a non-linear mathematical operation. A neuron receives inputs from one or more other neurons in the previous layer of the feedforward neural network, processes the inputs and then passes them on to other neurons in the next layer of the feedforward neural network. The neuron processes the inputs by computing a weighted sum of the inputs and then applying the non-linear mathematical operation, called an activation function, to the weighted sum before passing the result to the neurons in the next layer. The weights that the neuron applies to each received input represent parameters of the feedforward neural network. Similarly to a recurrent neural network, the parameters of a feedforward neural network are updated during training so that they best represent the data with respect to which the feedforward neural network is trained.

The feedforward neural network is trained using backpropagation, the most widely used algorithm in the fields of feedforward neural networks.

The backpropagation algorithm comprises a forward and a backward pass through all the layers of a neural network. In the forward pass, training instances are passed forward through all layers starting from the input layer, then through the hidden layers and finally to the output layer which produces an output for each training instance. At the end of the forward pass, a loss (or error) function L computes an error corresponding to each output. In the backward pass, the error is propagated backwards from the output layer to the input layer. During the backward pass, the weights of each neuron are updated in proportion to the neuron's contribution to the overall error calculated by computing the gradient of the loss function L with respect to that neuron. Therefore, during training, a network layer is updated once all the weights connecting neurons in that layer to neurons in the previous layer are updated. The parameter update is usually computed via an optimisation algorithm that minimises the loss function L. Usually iterative optimisation algorithms such as gradient descent or modified versions of gradient descent such as the Adam optimiser are used (see Kingma, Diederik P., and Jimmy Ba. "Adam: A method for stochastic optimization."arXiv preprint arXiv:1412.6980 (2014), incorporated by reference in this disclosure). Minimising the loss function L of a neural network is equivalent to driving the network outputs to be as close as possible to the target data. Once one forward pass and one backward pass is executed for all training instances, an epoch of training is completed. The number of epochs may be fixed or determined by a stopping criterion. In some embodiments, the Adam optimizer is used, and the learning rate is treated as a tunable hyperparameter of the neural network model.

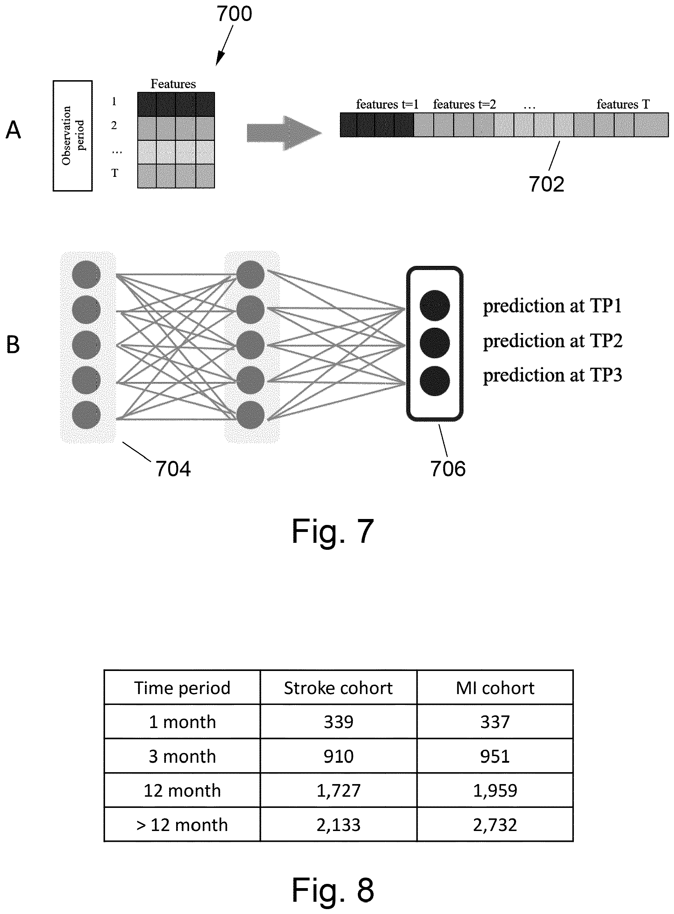

A feedforward neural network 704 is adapted to perform the prediction of disease onset for the multiple time periods defined by the target data by: 1) feeding in the input data as an aggregated feature vector 702 containing the data values for all the observation periods as shown in Figure 7A (rather than sequentially as in the case of a recurrent neural network 700), 2) adding a final fully connected layer 706 at the end of the feedforward neural network 704, as shown in Figure 7B, similar to the final fully connected layer 408 of the recurrent neural network model 400 shown in Figure 4, 3) using a binary cross entropy loss (as explained above with reference to the recurrent neural network model).

Subsequent to training the neural network (recurrent or otherwise) at step 104, at 106, the neural network model is used to predict the disease onset label in the form of the vector $y^P$. During the prediction stage, test input data comprising a set of data values of variables associated with one or more observation periods for a test patient *tp* is provided. Then, the input data provided is applied as an input to the neural network worked trained at step 104. The input is then processed by the trained neural network model through a forward pass in which the activations are computed and propagated through the hidden layers all the way to the final layer. The final layer computes the output as a vector$y^{tp}$ formed of a plurality of variables, each variable corresponding to a distinct time period from the two or more time periods. Each variable indicates the probability of the test patient having a diagnostic event of the disease that occurs within the time period that the variable corresponds. The vector $y^{tp}$ represents the predicted disease onset label for the test patient *tp*. Advantageously, the neural network model described above produces simultaneous predictions for two or more time periods, as discussed above.

*Detailed Example: Prediction of the onset of cardiovascular diseases*

In the detailed example described below, the neural network model of process 100 was applied to a dataset collected by an NHS Foundation Trust referred to here as OUH to predict disease onset for myocardial infarct (MI) patients and ischaemic stroke patients. The dataset contains two training datasets: one comprising 2732 target MI patients and 2732 control patients (OUH MI), and another comprising 2133 target stroke patients and 2133 control patients for the stroke cohort (OUH stroke).

The feature vectors in the OUH MI dataset contain variables that can be grouped into following categories: admission information (e.g. encounter type, discharge method, discharge destination), diagnoses (e.g. ICD-10 codes such as N185, L82X etc), procedures (e.g. OPCS-4 codes), laboratory test values (e.g. blood glucose, blood c reactive protein, blood sodium) and observations of vital signs (e.g. oxygen saturation, systolic blood pressure, tympanic temperature, respiratory rate). The feature vectors in the OUH stroke dataset contain variables that can be grouped into following categories: admission information, demographic information, diagnoses, procedures, laboratory test values, prescribed medications (e.g. BNF codes), observations of vital signs. Both datasets may also include risk scores derived from any of the categories listed, including Charlson's comorbidity index. When training a neural network model according to process 100, it is possible to use all or just subsets of the features in each category.

The neural network model used was a bidirectional GRU network. Using Bayesian optimization, two bidirectional GRU networks were optimized, one for predicting MI onset for the OUH MI training dataset and another for predicting ischaemic stroke onset for the OUH stroke training dataset. For both MI and stroke, four time periods were investigated: 1 month, 3 months, 12 months and >12 months since the last observation period for each patient. The table in Figure 8 shows the number of patients in the stroke and MI cohort for each of the 4 time periods investigated.

*Results for Detailed Example*

To provide a benchmark for the performance of the bidirectional GRU networks implemented according to process 100, logistic regression (LR) was used with three different settings: taking into account one observation period - LR(1), taking into account 50 observation periods - LR(50) and taking into account 100 observation periods - LR(100). In addition to this, single time period bidirectional GRU networks (ST-GRU) were trained separately, one for each of the four time periods. Both the multi time bidirectional GRU network (MT-GRU) and ST-GRU were trained for 100 epochs.

To provide a clinical benchmark, the QRISK score for the MI and stroke training datasets was also computed. The QRISK2 algorithm (see Hippisley-Cox, Julia, et al. "Predicting cardiovascular risk in England and Wales: prospective derivation and validation of QRISK2." Bmj 336.7659 (2008): 1475-1482, incorporated by reference in this disclosure) is the UK current primary care standard clinical assessment tool, which calculates a patient's risk of developing a cardiovascular disease over the next 10 years. The QRISK score is updated yearly and based on Cox proportional hazards models to estimate the coefficients and hazard rations associated with each potential risk factor. The patients with a score of 20% or higher are considered to be at high risk of developing cardiovascular diseases.

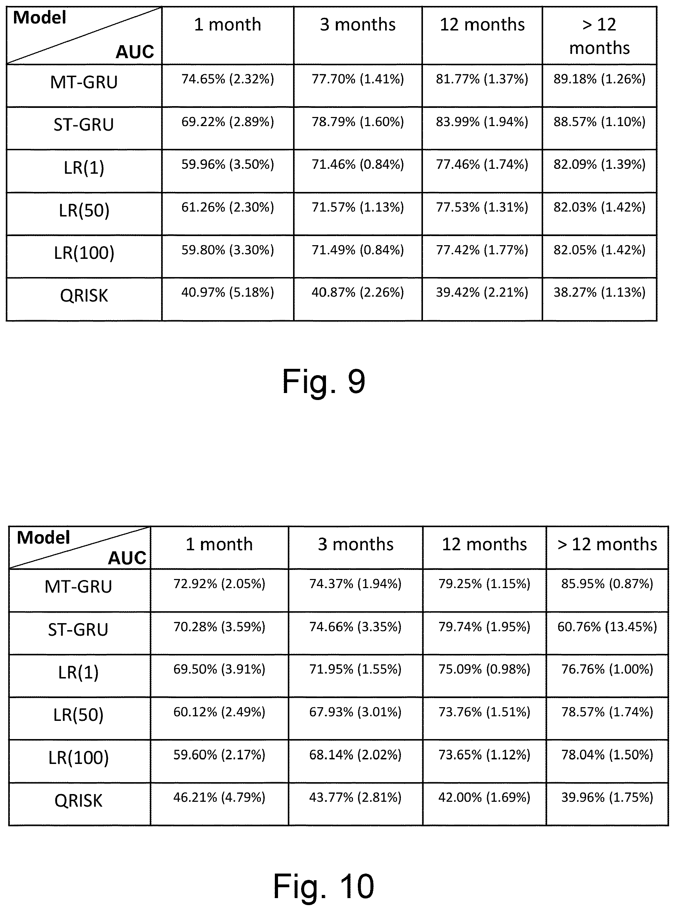

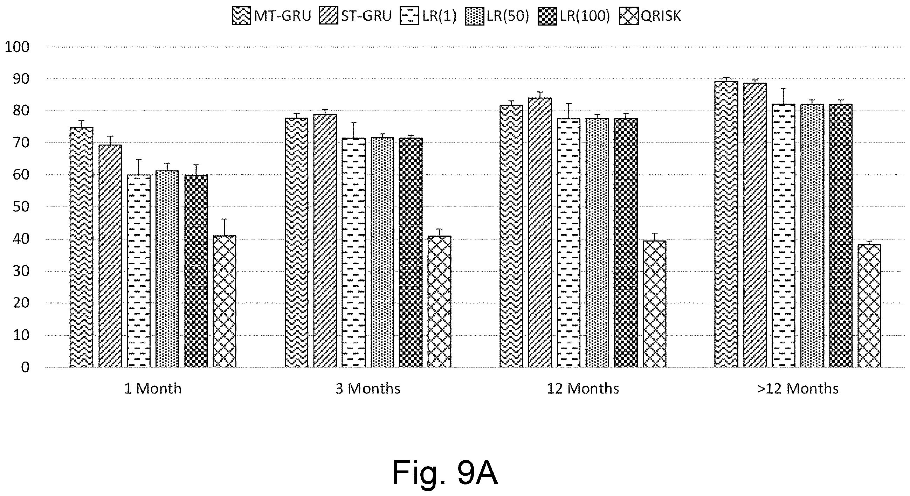

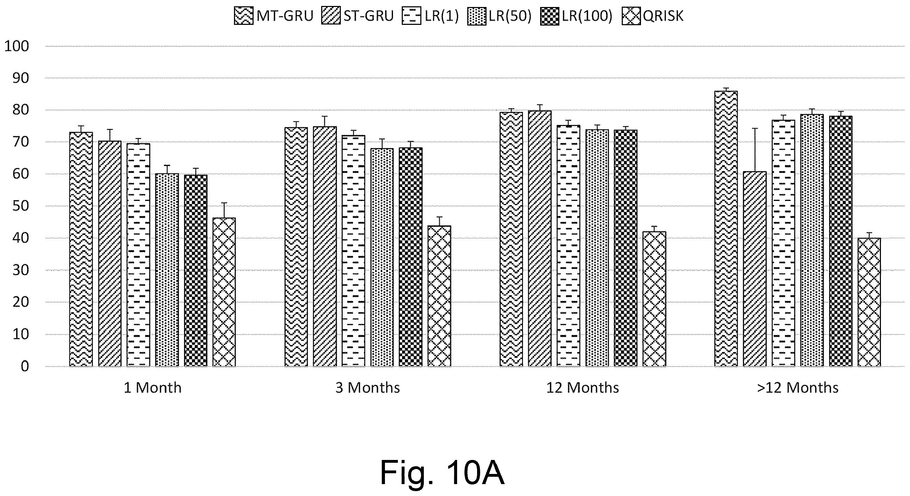

To assess the performance of the models, a 5-fold cross validation procedure was used. The tables in Figures 9 and 10 and bar charts in Figures 9A and 10B show the average area under the receiver operating characteristic curve (AUC) for the MT-GRU model and for the benchmark models. The numbers between brackets indicate the standard deviation based on the 5-fold cross validation. Figures 9 and 9A show the results for the MI training dataset, while Figures 10 and 10A show the results for the stroke training dataset.

Figures 9, 9A, 10 and 10A show that the neural network model outperforms the logistic regression and QRISK scores for all time periods predicted and for both MI (Figures 9 and 9A) and stroke (Figures 10 and 10A). When compared to the ST-GRU, Figures 9, 9A, 10 and 10A show that the MT-GRU provides a better prediction of disease onset for shorter time scales (1 month) and has a similar performance for longer time scales.

These results show that the MT-GRU approach is particularly beneficial over existing methods for shorter time scales (with smaller training sets - see Figure 8). While the ST-GRU network is trained on the small 1 month cohort of only 337/339 (MI/stroke) patients, the MT-GRU approach is trained on the whole training set, allowing the network to share information between different time periods, which leads to better training results. What's more, even for longer time periods (>12 months) when all training data is used by both MT-GRU and ST-GRU, the MT-GRU network has a better performance, especially for the stroke cohort in Figure 10 (AUC 85.95% versus AUC 60.76%). For the same time period, the MT-GRU network also improves the robustness (consistency) of the prediction of stroke onset, as shown by the standard deviations of the two models.

Since all networks in the specific example were trained for 100 epochs, it can be appreciated that the training to achieve better or comparable results is more efficient for the MT-GRU network than for the ST-GRU network. The total training for the MT-GRU network is 100 epochs, while the total training for the ST-GRU networks covering the same time periods is 400 epochs, 100 epochs for each time period. This results in a fourfold improvement in training epochs required, providing a corresponding reduction in time and computational resources. Specifically, the total compute time was 36 s (stroke) and 46 s (MI) for MT-GRU (one model) and 127 s (stroke) and 150 s (MI) for ST-GRU (four models), with all models running on the same hardware. In addition, the MT-GRU approach requires the training and optimization of only one neural network model, while the ST-GRU approach builds, trains and optimizes four separate network models. This also provides a corresponding reduction is computational resources (e.g. processor cycles, memory allocation), illustrating another benefit of the neural network model of process 100.

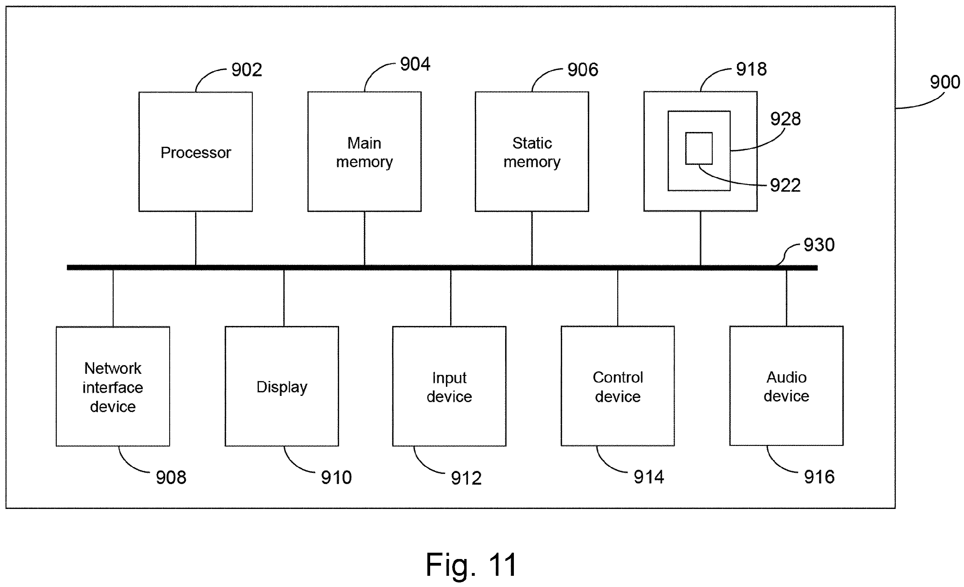

Figure 11 illustrates a block diagram of one implementation of a computing device 900 within which a set of instructions, for causing the computing device to perform any one or more of the methodologies discussed herein, may be executed. In alternative implementations, the computing device may be connected (e.g., networked) to other machines in a Local Area Network (LAN), an intranet, an extranet, or the Internet. The computing device may operate in the capacity of a server or a client machine in a client-server network environment, or as a peer machine in a peer-to-peer (or distributed) network environment. The computing device may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine. Further, while only a single computing device is illustrated, the term "computing device" shall also be taken to include any collection of machines (e.g., computers) that individually or jointly execute a set (or multiple sets) of instructions to perform any one or more of the methodologies discussed herein.

The example computing device 900 includes a processing device 902, a main memory 904 (e.g., read-only memory (ROM), flash memory, dynamic random access memory (DRAM) such as synchronous DRAM (SDRAM) or Rambus DRAM (RDRAM), etc.), a static memory 906 (e.g., flash memory, static random access memory (SRAM), etc.), and a secondary memory (e.g., a data storage device 918), which communicate with each other via a bus 930.

Processing device 902 represents one or more general-purpose processors such as a microprocessor, central processing unit, or the like. More particularly, the processing device 902 may be a complex instruction set computing (CISC) microprocessor, reduced instruction set computing (RISC) microprocessor, very long instruction word (VLIW) microprocessor, processor implementing other instruction sets, or processors implementing a combination of instruction sets. Processing device 902 may also be one or more special-purpose processing devices such as an application specific integrated circuit (ASIC), a field programmable gate array (FPGA), a digital signal processor (DSP), network processor, or the like. Processing device 902 is configured to execute the processing logic (instructions 922) for performing the operations and steps discussed herein.

The computing device 900 may further include a network interface device 908. The computing device 900 also may include a video display unit 910 (e.g., a liquid crystal display (LCD) or a cathode ray tube (CRT)), an alphanumeric input device 912 (e.g., a keyboard or touchscreen), a cursor control device 914 (e.g., a mouse or touchscreen), and an audio device 916 (e.g., a speaker).

The data storage device 918 may include one or more machine-readable storage media (or more specifically one or more non-transitory computer-readable storage media) 928 on which is stored one or more sets of instructions 922 embodying any one or more of the methodologies or functions described herein. The instructions 922 may also reside, completely or at least partially, within the main memory 904 and/or within the processing device 902 during execution thereof by the computer system 900, the main memory 904 and the processing device 902 also constituting computer-readable storage media.

The various methods described above may be implemented by a computer program. The computer program may include computer code arranged to instruct a computer to perform the functions of one or more of the various methods described above. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may be or include a special-purpose processor, such as a field programmable gate array (FPGA) or an ASIC. A hardware component may also include programmable logic or circuitry that is temporarily configured by software to perform certain operations.

Accordingly, the phrase "hardware component" should be understood to encompass a tangible entity that may be physically constructed, permanently configured (e.g., hardwired), or temporarily configured (e.g., programmed) to operate in a certain manner or to perform certain operations described herein.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as "receiving", "determining", "comparing ", "enabling", "maintaining," "identifying", "providing", "applying", "training" or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

**Claims**

1. A computer-implemented method of training a neural network to predict a disease onset label corresponding to a diagnostic event of a disease, the method comprising:

   providing training data comprising input data and target data for a plurality of patients comprising target patients having a diagnostic event of the disease and control patients not having a diagnostic event of the disease, the input data comprising a set of data values of respective variables for each of a plurality of patients, wherein the set comprises for each variable one or more data values associated with respective one or more observation periods, and the target data comprising a disease onset label for each of the plurality patients, wherein each disease onset label indicates whether a respective patient has a diagnostic event of the disease that occurs within each of two or more time periods; and
   training, using the training data, a neural network model to predict a corresponding disease onset label from the respective variables associated with one or more observation periods, the disease onset label indicating whether a respective patient has a diagnostic event of the disease that occurs within each of the two or more time periods.

2. A computer-implemented method of predicting a disease onset label corresponding to a diagnostic event of a disease, the method comprising:

   providing test input data comprising a set of data values of respective variables associated with one or more observation periods for a test patient;
   applying the test input data as an input to a neural network trained according to claim 1 to predict a disease onset label indicating the likelihood of the test patient having a diagnostic event of the disease that occurs within each of the two or more time periods.

3. The method of claim 1 or 2, wherein the observation period is a day.

4. The method of any preceding claim, wherein the neural network is a recurrent neural network.

5. The method of claim 4, wherein training the recurrent neural network comprises applying the data values for the respective variables for each of the plurality of patients as input to the recurrent neural network sequentially, one set of data values for the respective variables associated with one observation period at a time, and reducing a loss across the data values.

6. The method of claim 5, wherein the recurrent neural network is used to predict a disease onset label for a respective patient by applying the data values for the respective variables for the respective patient as input to the recurrent neural network sequentially to produce an output for the respective patient.

7. The method of any one of claims 4 to 6, wherein the recurrent neural network is a gated recurrent unit (GRU) neural network.

8. The method of claim 1, wherein providing the training data comprises obtaining the input data from health records.

9. The method of claim 8, wherein the training data comprises two or more categories of the categories of: demographic information; admission information; diagnoses; procedures; laboratory test values; prescribed medications; observations of vital signs; and anthropomorphic measurements.

10. The method of any of the preceding claim, wherein the two or more time periods start at a common reference point and have different end points.

11. The method of any preceding claim, wherein the disease is a cardiovascular disease.

12. The method of claim 11, wherein the cardiovascular disease is ischaemic stroke or myocardial infarct.

13. One or more computer-readable media encoding computer instruction that, when executed on a computing device, implement a method according to any one of the preceding claims.

14. A system comprising a computer processor and a memory storing computer instruction that, when executed on the processor, implement a method according to any one of claims 1-12.

100

```
┌─────────────────────┐
│ receive and preprocess │── 102
│   training data        │
└─────────────────────┘
           │
           ▼
┌─────────────────────┐
│ use training data to train a │── 104
│ neural network model to      │
│ predict disease onset        │
└─────────────────────┘
           │
           ▼
┌─────────────────────┐
│ predict disease onset for │── 106
│ two or more time periods   │
│ for a test patient         │
└─────────────────────┘
```

# Fig. 1

EP 3 920 191 A1

Variables

| | | | | | |
|---|---|---|---|---|---|
| $t$ | $X_{t1}$ | $X_{t2}$ | ... | $X_{t(n-1)}$ | $X_{1n}$ |
| $t-1$ | $X_{(t-1)1}$ | $X_{(t-1)2}$ | ... | $X_{(t-1)(n-1)}$ | $X_{(t-1)n}$ |

| | | | | | |
|---|---|---|---|---|---|
| $2$ | $X_{21}$ | $X_{22}$ | ... | $X_{2(n-1)}$ | $X_{2n}$ |
| $1$ | $X_{11}$ | $X_{12}$ | ... | $X_{1(n-1)}$ | $X_{1n}$ |

Observation period

Input data $x_{1:t}^{P}$

200

| TP1 | TP2 | TP3 |
|---|---|---|
| 0 | 1 | 1 |

204

Target data $y^{p}$

202

Fig. 2

Fig. 3

Fig. 4

Fig. 5

Fig. 6

700

Features

A

Observation period

1
2
...
T

features t=1    features t=2    ...    features T

702

B

prediction at TP1

prediction at TP2

prediction at TP3

704

706

# Fig. 7

| Time period | Stroke cohort | MI cohort |
|-------------|---------------|-----------|
| 1 month | 339 | 337 |
| 3 month | 910 | 951 |
| 12 month | 1,727 | 1,959 |
| > 12 month | 2,133 | 2,732 |

# Fig. 8

| Model ╱ AUC | 1 month | 3 months | 12 months | > 12 months |
|---|---|---|---|---|
| MT-GRU | 74.65% (2.32%) | 77.70% (1.41%) | 81.77% (1.37%) | 89.18% (1.26%) |
| ST-GRU | 69.22% (2.89%) | 78.79% (1.60%) | 83.99% (1.94%) | 88.57% (1.10%) |
| LR(1) | 59.96% (3.50%) | 71.46% (0.84%) | 77.46% (1.74%) | 82.09% (1.39%) |
| LR(50) | 61.26% (2.30%) | 71.57% (1.13%) | 77.53% (1.31%) | 82.03% (1.42%) |
| LR(100) | 59.80% (3.30%) | 71.49% (0.84%) | 77.42% (1.77%) | 82.05% (1.42%) |
| QRISK | 40.97% (5.18%) | 40.87% (2.26%) | 39.42% (2.21%) | 38.27% (1.13%) |

# Fig. 9

| Model ╱ AUC | 1 month | 3 months | 12 months | > 12 months |
|---|---|---|---|---|
| MT-GRU | 72.92% (2.05%) | 74.37% (1.94%) | 79.25% (1.15%) | 85.95% (0.87%) |
| ST-GRU | 70.28% (3.59%) | 74.66% (3.35%) | 79.74% (1.95%) | 60.76% (13.45%) |
| LR(1) | 69.50% (3.91%) | 71.95% (1.55%) | 75.09% (0.98%) | 76.76% (1.00%) |
| LR(50) | 60.12% (2.49%) | 67.93% (3.01%) | 73.76% (1.51%) | 78.57% (1.74%) |
| LR(100) | 59.60% (2.17%) | 68.14% (2.02%) | 73.65% (1.12%) | 78.04% (1.50%) |
| QRISK | 46.21% (4.79%) | 43.77% (2.81%) | 42.00% (1.69%) | 39.96% (1.75%) |

# Fig. 10

Fig. 9A

Fig. 10A

Fig. 11

Europäisches Patentamt

European Patent Office

Office européen des brevets

# EUROPEAN SEARCH REPORT

Application Number

EP 20 17 8631

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SUNIL MALLYA ET AL: "Effectiveness of LSTMs in Predicting Congestive Heart Failure Onset", ARXIV.ORG, CORNELL UNIVERSITY LIBRARY, 201 OLIN LIBRARY CORNELL UNIVERSITY ITHACA, NY 14853, 7 February 2019 (2019-02-07), XP081026257, * abstract; figures 1-3, 5 * * page 1 - page 7 * | 1-14 | INV. G16H50/20 G16H50/30 |
| A | SIERRA-SOSA DANIEL ET AL: "Scalable Healthcare Assessment for Diabetic Patients Using Deep Learning on Multiple GPUs", IEEE TRANSACTIONS ON INDUSTRIAL INFORMATICS, IEEE SERVICE CENTER, NEW YORK, NY, US, vol. 15, no. 10, 1 October 2019 (2019-10-01), pages 5682-5689, XP011749309, ISSN: 1551-3203, DOI: 10.1109/TII.2019.2919168 [retrieved on 2019-10-03] * the whole document * | 1-14 | |
| A | DUAN HUILONG ET AL: "On Clinical Event Prediction in Patient Treatment Trajectory Using Longitudinal Electronic Health Records", IEEE JOURNAL OF BIOMEDICAL AND HEALTH INFORMATICS, IEEE, PISCATAWAY, NJ, USA, vol. 24, no. 7, 24 December 2019 (2019-12-24), pages 2053-2063, XP011796245, ISSN: 2168-2194, DOI: 10.1109/JBHI.2019.2962079 [retrieved on 2020-07-01] * the whole document * | 1-14 | TECHNICAL FIELDS SEARCHED (IPC) G16H |

The present search report has been drawn up for all claims

| Place of search | Date of completion of the search | Examiner |
|---|---|---|
| Munich | 20 November 2020 | Kutzarova, Iskrena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GOLDSTEIN ; BENJAMIN A. et al.** Opportunities and challenges in developing risk prediction models with electronic health records data: a systematic review. *Journal of the American Medical Informatics Association,* 2017, vol. 24.1, 198-208

- **QUAN ; HUDE et al.** Updating and validating the Charlson comorbidity index and score for risk adjustment in hospital discharge abstracts using data from 6 countries. *American journal of epidemiology,* 2011, vol. 173.6, 676-682

- **KINGMA ; DIEDERIK P. ; JIMMY BA.** *Adam: A method for stochastic optimization."arXiv preprint arXiv,* 2014

- **HIPPISLEY-COX ; JULIA et al.** Predicting cardiovascular risk in England and Wales: prospective derivation and validation of QRISK2. *Bmj,* 2008, vol. 336.7659, 1475-1482